# EUROPEAN PATENT APPLICATION

(11) **EP 2 711 430 A1**
(43) Date of publication of application: **26.03.2014**
(21) Application number: 12185629.8
(22) Date of filing: 24.09.2012
(51) Int. Cl.: C12Q 1/68

(54) **MicroRNA based method for diagnosis of colorectal tumors and of metastasis**

(71) Applicant: Max-Planck-Gesellschaft zur Förderung der Wissenschaften e.V., 80539 München (DE)
(72) Inventor: Schweiger, Michal-Ruth, Dr. Dr., 14167 Berlin (DE); Lehrach, Hans, Prof. Dr., 14129 Berlin (DE)
(74) Representative: Fanelli Haag Kilger PLLC

(57) **Abstract**

The invention encompasses the identification and selection of novel miRNAs as biomarkers so as to provide a method for the diagnosis of colorectal cancer. The miRNAs identified are differentially expressed in subjects with colorectal cancer compared to subjects without colorectal cancer. Further, some of the identified miRNAs were found to play a critical role for the progression of colorectal cancer into metastasis and, thus, facilitate a differential diagnosis between tumor and metastasis. Nucleic acids which are capable of specifically detecting the miRNAs identified are also encompassed within the scope of the invention as are sets of said nucleic acids, which are particularly suited for being used in multiplex RT-PCR or in array techniques. Further encompassed are compositions and kits containing said nucleic acids and nucleic acids for use in diagnosing colorectal cancer.

## Description

### FIELD OF THE INVENTION

The present invention is in the field of medicine, more in particular in the field of molecular medicine and diagnostics. The invention relates in particular to the diagnosis of colorectal tumors and metastasis.

### BACKGROUND

MicroRNAs are evolutionarily conserved, small (20-25 nucleotides) non-protein-coding molecules that regulate gene expression at the post-transcriptional level. These single stranded RNAs participate in the regulation of various cellular processes, such as cell differentiation, cell cycle progression, metabolism and apoptosis. Here they influence, among other biological systems, immunity and cancer.

The regulatory potential of microRNAs is due to the fact that each microRNA targets multiple mRNAs and one mRNA can be regulated by different microRNAs. Through base pairing with the 3'UTR of mRNA targets they negatively regulate gene expression at the level of translation. The transcription of the primary microRNA, which contains a local fold back structure, is mediated predominantly by RNA Polymerase II in the nucleus and is processed by the Drosha/Pasha nuclease complex. The resulting hairpin structure, called pre-miRNA, is transported to the cytoplasm, where it undergoes further processing by Dicer, liberating a ∼22 bp double stranded microRNA duplex structure that consists of the mature and the complementary mature star sequence. The mature microRNA is incorporated into the miRISC complex (miRNA-containing RNA-induced silencing complex) and binds preferentially to the 3'UTR of mRNA target molecules.

With the initial discovery of microRNAs in 1993, a new class of gene regulation was found. Ambros, Lee and colleagues revealed that the gene lin-4 is transcribed into a 22 nucleotide RNA molecule, which inhibits the protein synthesis of Lin-14 by nearly perfect complementary binding to its 3' untranslated region (3'UTR). This process affects temporal progression of cell differentiation in the nematode Caenorhabditis elegans. At the moment, more than 1000 (1049) microRNA sequences have been identified in the human genome (miRBase v16, 2010). It is predicted that approximately 3% of the human genome encodes for microRNAs and ∼30% of the protein-encoding genes are regulated by miRNAs. In 2002 Calin et al. were the first who described the association between microRNAs and cancer. They demonstrated that in over 65% of B cell chronic lymphocytic leukemia (B-CLL) patients, the miRNA genes miR-15 and miR-16 are located within a deletion on chromosome 13, which is the most frequent abnormality in B-CLL. In these cases both miRNAs are not expressed and lead to B-CLL.

According to the World Health Organization (WHO) colorectal cancer is the third most common cancer worldwide and a major cause of cancer mortality with an incidence of approximately 1 million cases). At early stages of CRC a curative treatment is achieved by surgical resection or by chemotherapy which is often applied in an adjuvant or neo adjuvant manner.

Once the tumor metastasizes it is incurable. It is therefore important to detect CRC as early as possible and biomarkers for screening purposes would be highly desirable.

### DESCRIPTION OF THE INVENTION

The invention encompasses the identification and selection of novel miRNAs as biomarkers so as to provide a method for the diagnosis of colorectal cancer. The miRNAs identified are differentially expressed in subjects with colorectal cancer compared to subjects without colorectal cancer. Further, some of the identified miRNAs were found to play a critical role for the progression of colorectal cancer into metastasis and, thus, facilitate a differential diagnosis between tumor and metastasis.

Nucleic acids which are capable of specifically detecting the miRNAs identified are also encompassed within the scope of the invention as are sets of said nucleic acids, which are particularly suited for being used in multiplex RT-PCR or in array techniques. Further encompassed are compositions and kits containing said nucleic acids and nucleic acids for use in diagnosing colorectal cancer.

The following detailed description of the invention refers, in part, to the accompanying drawings and does not limit the invention.

### DEFINITIONS

The following definitions are provided for specific terms which are used in the following.

As used herein, the term "biomarker" refers to a miRNA that is differentially expressed, i.e. upregulated or downregulated, wherein the status (up-/downregulation) of said biomarker can be used for diagnosing colorectal cancer or a stage of colorectal cancer as compared with those not having colorectal cancer or can be used for differentially diagnosing colorectal cancer.

As used herein, the term "composition" refers to any mixture. It can be a solution, a suspension, liquid, powder, a paste, aqueous, non-aqueous or any combination thereof.

As used herein, the term "diagnosis" refers to the identification of the disease (colorectal cancer) at any stage of its development, and also includes the determination of predisposition of a subject to develop the disease. In a preferred embodiment of the invention, diagnosis of colorectal cancer occurs prior to the manifestation of symptoms. Subjects with a higher risk of developing the disease are of particular concern. The diagnostic method of the invention also allows confirmation of colorectal cancer in a subject suspected of having colorectal cancer. "Differential diagnosis" refers to differentiating between tumour and metastasis, thereby facilitating the differentiation between an individual having metastasis-free colorectal cancer and an individual having metastatic colorectal cancer.

As used herein, the term "differential expression" refers to a difference in the level of expression of the miRNA of one or more biomarkers, as measured by the amount of miRNA, in one sample as compared with the level of expression of the same one or more biomarkers of the invention in a second sample or with regard to a predetermined reference value. Differential expression can be determined, e.g. by array hybridization, next generation sequencing, RT-PCR and as would be understood by a person skilled in the art.

As used herein, the terms "hybridizing to" and "hybridization" are interchangeable used with the terms "specific for" and "specifically binding" and refer to the sequence specific non-covalent binding interactions with a complementary nucleic acid, for example, interactions between a target nucleic acid sequence and a target specific nucleic acid primer or probe. In a preferred embodiment a nucleic acid, which hybridizes is one which hybridizes with a selectivity of greater than 70%, greater than 80%, greater than 90% and most preferably of 100% (i.e. cross hybridization with other DNA species preferably occurs at less than 30%, less than 20%, less than 10%). As would be understood to a person skilled in the art, a nucleic acid, which "hybridizes" to the DNA product of a genomic region of the invention can be determined taking into account the length and composition.

As used herein, a "kit" is a packaged combination optionally including instructions for use of the combination and/or other reactions and components for such use.

The term nucleic acid is here used in its broadest sense and comprises ribonucleic acids (RNA) and deoxyribonucleic acids (DNA) from all possible sources, in all lengths and configurations, such as double-stranded, single-stranded, circular, linear or branched. All sub-units and sub-types are also comprised, such as monomeric nucleotides, oligomers, plasmids, viral and bacterial nucleic acids, as well as genomic and non-genomic DNA and RNA from animal and plant cells or other eukaryotes or prokaryotes, mRNA (messenger RNA) in processed and unprocessed form, tRNA (transfer RNA), hn-RNA (heterogeneous nuclear RNA), rRNA (ribosomal RNA), LNA (locked nucleic acid), mtRNA (mitochondrial), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or as well as all other conceivable nucleic acids.

The term "primer", as used herein, refers to an nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides. Oligonucleotide primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing oligonucleotides on a modified solid support is described in U.S. Pat. No. 4,458,006, which is hereby incorporated by reference.

As used herein, the term "probe" means nucleic acid and analogs thereof and refers to a range of chemical species that is substantially complementary to a specific nucleic acid sequence. The probe or the target sequences may be single- or double-stranded DNA. A probe is at least 8 nucleotides in length and less than the length of a complete polynucleotide target sequence. A probe may be 8 nt to 100 nt, preferably 10 nt to 50 nt, more preferably 12 nt to 40 nt, even more preferably 14 nt to 30, most preferably 15 nt to 25 nt in length. Probes can include nucleic acids modified so as to have a tag which is detectable by fluorescence, chemiluminescence and the like ("labelled probe"). The labelled probe can also be modified so as to have both a detectable tag and a quencher molecule, for example Taqman® and Molecular Beacon® probes. Suitable probes include the LightCycler probe (Roche), the TaqMan probe (Life Technologies), a molecular beacon probe, a Scorpion primer, a Sunrise primer, a LUX primer and an Amplifluor primer. Probes may be situated between the primer pairs.

The term "sample" is used herein to refer to colorectal tissue, blood, urine, semen, colorectal secretions or isolated colorectal cells originating from a subject, preferably from colorectal tissue, colorectal secretions or isolated colorectal cells, most preferably to colorectal tissue.

As used herein, the terms "subject" and "patient" are used interchangeably to refer to an animal (e.g., a mammal, a fish, an amphibian, a reptile, a bird and an insect). In a specific embodiment, a subject is a mammal (e.g., a non-human mammal and a human). In another embodiment, a subject is a primate (e.g., a chimpanzee and a human). In another embodiment, a subject is a human. In another embodiment, the subject is a male human with or without colorectal cancer.

### DETAILED DESCRIPTION OF THE INVENTION

To address the need in the art for an enhanced diagnosis of colorectal cancer, the peculiarities of the miRNA expression profile across the whole genome of colorectal cancer positive samples were examined in comparison to colorectal cancer negative samples. The inventors found miRNAs, that are subject to a differential expression level. Therefore, the invention teaches the analysis of those miRNAs that are differentially expressed in samples from patients having colorectal cancer. Most astonishingly, the inventors found miRNAs, which facilitate a differential diagnosis between tumour and metastatic samples.

The herein disclosed methods are also particularly useful for early diagnosis of colorectal cancer. The method is useful for further diagnosing patients having symptoms associated with colorectal cancer. The method of the present invention can further be of particular use with patients having an enhanced risk of developing colorectal cancer (e.g., patients having a familial history of colorectal cancer and patients identified as having a mutant oncogene). The method of the present invention may further be of particular use in monitoring the efficacy of treatment of a colorectal cancer patient (e.g. the efficacy of chemotherapy).

While the herein disclosed biomarkers are particularly useful in diagnosing colorectal cancer, the inventors surprisingly found that said biomarkers are equally useful in diagnosing cancer in general. Such cancers include cancers of, for example, adipose tissue, brain, breast, colon, endometrium, kidney, liver, lung, lymph node, muscle, ovary, pancreas, prostate, stomach, testis and thyroid gland. Accordingly, the means and methods of the herein disclosed invention are not limited to the diagnosis of colorectal cancer, but can be also applied to any cancer, preferably selected from the list of adipose tissue, brain, breast, colon, endometrium, kidney, liver, lung, lymph node, muscle, ovary, pancreas, prostate, stomach, testis and thyroid gland.

The present invention contemplates a method for differential diagnosis of colon cancer in a patient, comprising the steps of, determining in a sample of a subject the amount of one or more of the micro RNAs (miRNA) selected from the list of Table 1, comparing the amount of miRNA determined to a predetermined reference value, wherein if the miRNA is, in comparison to said reference value, either down-regulated or up-regulated as designated in Table 1, the sample is designated as metastatic.

**Table 1:**

| **microRNA name** | **regulation in metastatic sample as compared to reference value** |
|---|---|
| miR-194-1 | down-regulated |
| miR-202 | down-regulated |
| miR-551b | down-regulated |
| miR-129-2 | down-regulated |
| miR-658 | down-regulated |
| miR-497 | down-regulated |
| miR-3152 | down-regulated |
| miR-378c | down-regulated |
| miR-605 | down-regulated |
| miR-559 | down-regulated |
| miR-184 | up-regulated |
| miR-1248 | up-regulated |
| miR-450b | up-regulated |
| miR-1975 | up-regulated |
| miR-3122 | up-regulated |
| miR-542 | up-regulated |
| miR-4286 | up-regulated |
| miR-503 | up-regulated |
| miR-877 | up-regulated |
| miR-1292 | up-regulated |

The herein disclosed methods make use of determining the expression level of miRNAs. The most widely used miRNA detection method is Northern blotting, which is considered as the standard of miRNA detection methods. This method, however, is time-consuming and has low sensitivity. RT-PCR is the most sensitive method thus far. The state-of-the-art microarrays are unrivalled in terms of parallelism and throughput. Preferred in the context of the present invention are miRNA array techniques and RT-PCR, mostly preferred RT-PCR.

The amount of the same miRNA present in a benign sample may serve as a predetermined reference in the case of a differential diagnosis. The reference value may be deduced from literature. The reference value may have alternatively been determined in a separate experiment, preferably by using the same experimental setting as used in the inventive method.

As will be understood by the person skilled in the art, comparing the amount of miRNA in a sample to a reference value relates to a correlation that serves as a basis for diagnosis. For example, it preferably relates to the ratio of the amount of miRNA in a sample to a predetermined reference value which corresponds to the amount of miRNA of a reference sample.

The present invention further contemplates a method for diagnosis of colon cancer in a patient, comprising the following steps of, determining in a sample of a subject the amount of one or more of the micro RNAs (miRNA) selected from the list of Table 2, comparing the amount of miRNA determined to a predetermined reference value, wherein if the miRNA is, in comparison to said reference value, either down-regulated or up-regulated as designated in Table 2, the sample is designated as cancerous.

**Table 2:**

| **microRNA name** | **regulation in cancerous sample as compared to reference value** |
|---|---|
| Hsa-mir-1224 | down-regulated |
| Hsa-mir-202 | down-regulated |
| Hsa-mir-147b | down-regulated |
| Hsa-mir-3154 | down-regulated |
| Hsa-mir-3065 | down-regulated |
| Hsa-mir-3150 | down-regulated |
| Hsa-mir-3152 | down-regulated |
| Hsa-mir-497 | down-regulated |
| Hsa-mir-585 | down-regulated |
| Hsa-mir-378b | down-regulated |
| Hsa-mir-3175 | up-regulated |
| Hsa-mir-450a2 | up-regulated |
| Hsa-mir-449b | up-regulated |
| Hsa-mir-1286 | up-regulated |
| Hsa-mir-3117 | up-regulated |
| Hsa-mir-1323 | up-regulated |
| Hsa-mir-450a-1 | up-regulated |
| Hsa-mir-323b | up-regulated |
| Hsa-mir-450b | up-regulated |
| Hsa-mir-452 | up-regulated |

In the case of a (non-differential) diagnosis, the reference value represents the amount of the miRNA of the same biomarker present in benign tissue. The reference value may be taken from literature or may have been determined in a separate experiment as outlined above.

In one embodiment of the method, the sample comprises cells obtained from a patient. Any sample which includes cells originating from the colon may be used. The cells may be found in a colorectal tissue sample collected, for example, by a colorectal tissue biopsy or histology section, or the metastasis tissue including among others liver, lung or bone marrow biopsy if metastatic spreading has occurred. In another embodiment, the patient sample is a colorectal-associated body fluid. Such fluids include, for example, lymph, blood, urine, semen, preferably from colorectal secretions, isolated colorectal cells, and most preferably from colorectal tissue. For metastatic cancer the fluids may include ascites or material from a bronchoalveolar lavage. From the samples cellular or cell free miRNA is isolated using standard molecular biological technologies and then forwarded to the analysis method.

To translate the raw data generated by the detection assay (i.e. expression level) into data of predictive value for a clinician, a computer-based analysis program can be used.

The profile data may be prepared in a format suitable for interpretation by a treating clinician. For example, rather than providing raw expression level, the prepared format may represent a diagnosis or risk assessment (e.g. likelihood of cancer being present or the subtype of cancer) for the subject, along with recommendations for particular treatment options.

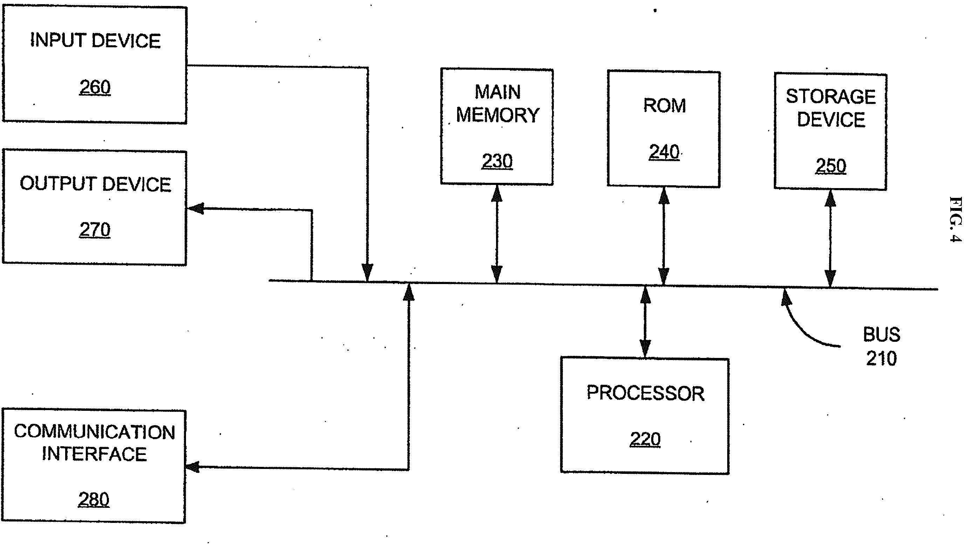

In one embodiment of the present invention, a computing device comprising a client or server component may be utilized. Figure 4 is an exemplary diagram of a client/server component, which may include a bus **210,** a processor **220,** a main memory **230,** a read only memory (ROM) **240,** a storage device **250,** an input device **260,** an output device **270,** and a communication interface **280.** Bus 210 may include a path that permits communication among the elements of the client/server component.

Processor **220** may include a conventional processor or microprocessor, or another type of processing logic that interprets and executes instructions. Main memory **230** may include a random access memory (RAM) or another type of dynamic storage device that stores information and instructions for execution by processor 220. ROM **240** may include a conventional ROM device or another type of static storage device that stores static information and instructions for use by processor **220.** Storage device **250** may include a magnetic and/or optical recording medium and its corresponding drive.

Input device 260 may include a conventional mechanism that permits an operator to input information to the client/server component, such as a keyboard, a mouse, a pen, voice recognition and/or biometric mechanisms, etc. Output device **270** may include a conventional mechanism that outputs information to the operator, including a display, a printer, a speaker, etc. Communication interface **280** may include any transceiver-like mechanism that enables the client/server component to communicate with other devices and/or systems. For example, communication interface **280** may include mechanisms for communicating with another device or system via a network.

As will be described in detail below, the client/server component, consistent with the principles of the invention, may perform certain measurement determinations of expression, calculations of expression level, and/or correlation operations relating to the diagnosis of colorectal cancer. It may further optionally output the presentation of status results as a result of the processing operations conducted. The client/server component may perform these operations in response to processor **220** executing software instructions contained in a computer-readable medium, such as memory **230.** A computer-readable medium may be defined as a physical or logical memory device and/or carrier wave.

The software instructions may be read into memory **230** from another computer-readable medium, such as data storage device **250,** or from another device via communication interface **280.** The software instructions contained in memory **230** may cause processor **220** to perform processes that will be described later. Alternatively, hardwired circuitry may be used in place of or in combination with software instructions to implement processes consistent with the principles of the invention. Thus, implementations consistent with the principles of the invention are not limited to any specific combination of hardware circuitry and software.

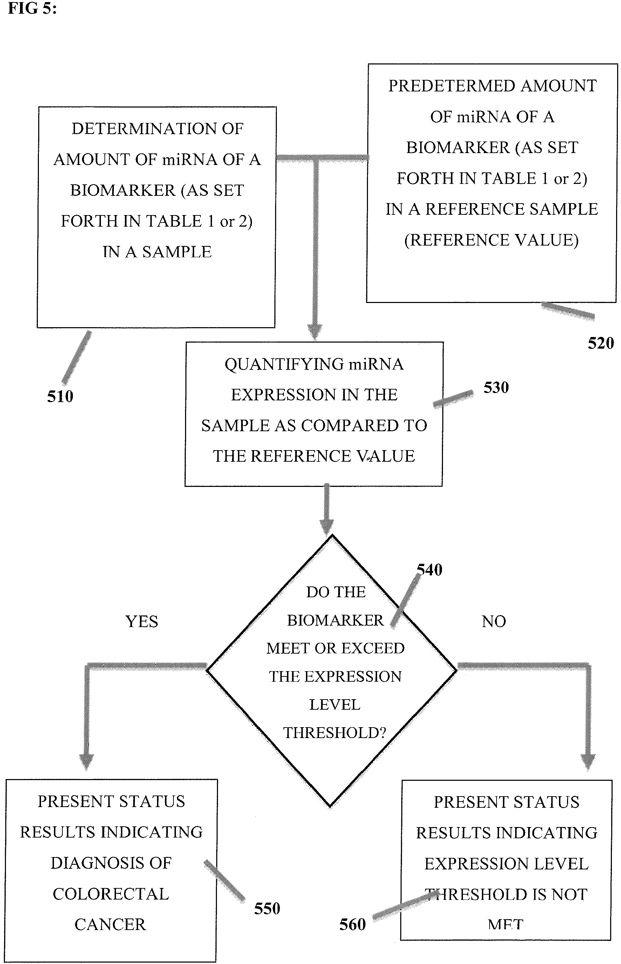

FIG. 5 is a flowchart of exemplary processing of expression level for miRNAs present in biological samples according to an implementation consistent with the principles of the present invention. Processing may begin with determining the amount of miRNA of a sample for a biomarker **510** as compared to a predetermined reference value **520.** The processor may then quantify the expression level **530,** as described above, e.g. as the ratio of the amount of miRNA present in said sample to the predetermined reference value, which corresponds to the amount of miRNA present in a reference sample for the same biomarker(s). The expression level may then be evaluated either via a computing device **540** or by human analysis to determine if the biomarker(s) meet or exceed a predetermined expression threshold. If the threshold is met or exceeded, the computing device may then, optionally, present a status result indicating a positive diagnosis of colorectal cancer **550.** Alternatively, if the threshold is not met, then the computing device may, optionally, present a status result indicating that the threshold is not satisfied **560.**

In the case of a differential diagnosis the computing device may, optionally, present a status result indicating a metastasis **550** or a tumour **560,** depending on whether the threshold is met or not.

It is noted that the output displaying results may differ depending on the desired presentation of results. For example, the output may be quantitative in nature, e.g., displaying the measurement values of the biomarker in relation to the predetermined reference value. The output may be qualitative, *e.g.,* the display of a color or notation indicating a positive result for colorectal cancer, or a negative results for colorectal cancer, or where applicable a positive result for tumour or metastasis, as the case may be. Notably, this process may be repeated multiple times using different miRNAs, as set forth in Tables 1 and 2. The computing device may alternatively be programmed to permit the analysis of more than one miRNA at one time.

The invention also relates to a nucleic acid that is able to specifically detect one of the miRNAs disclosed herein. That means that the nucleic acid may hybridize under stringent conditions to a sequence corresponding to the miRNA, or to the reverse complement thereof. The sequence may be DNA or RNA. In a preferred embodiment, the miRNA is reverse transcribed into cDNA for which the nucleic acid is specific.

In one embodiment the nucleic acid is 15 to 30 nt in length. In a preferred embodiment said nucleic acid is 15 to 25 nt in length.

In one embodiment said nucleic acid is a primer. The inventive primers being able to specifically detect one of the inventive miRNAs can be used for the analysis methods of the miRNA expression. Accordingly, they are used for amplification of a sequence comprising the miRNA or parts thereof in the inventive method for the diagnosis of colorectal cancer. Within the context of the invention, the primers selectively hybridize under stringent conditions to a RNA or DNA sequence corresponding to the miRNA, or to the reverse complement thereof, as defined above.

The expression level may be determined by a probe. Hence, in another embodiment said nucleic acid is a probe. The inventive probes are able to specifically detect a miRNA. Within the context of the invention, the probes selectively hybridize under stringent conditions to a RNA or DNA sequence corresponding to the miRNA, or to the reverse complement thereof, as defined above.

The present invention further relates to a set of the herein disclosed nucleic acids, each of which is able to specifically detect a (different) miRNA disclosed herein. A set of nucleic acids means at least two nucleic acids, preferably, three, four, five, six, seven, eight, nine or even ten and more nucleic acids. A set of nucleic acids is particularly useful for increasing the confidence of diagnosis. A set of nucleic acids is preferably used in a multiplex fashion, such as multiplex RT-PCR or micro-array techniques.

The nucleic acid for performing the method according to the invention is advantageously formulated in a stable composition. Accordingly, the present invention relates to a composition for the diagnosis of colorectal cancer comprising one of the herein disclosed nucleic acids or a set of nucleic acids as defined above. The composition may include other substances known to the skilled person, such as stabilizers.

Further encompassed by the present invention is a kit for the diagnosis of colorectal cancer comprising a nucleic acid as defined herein, or a set of nucleic acids as defined herein, or a composition as defined herein.

The kit may comprise a container for a primer or a set of primers as described herein. The kit may also comprise one or more probes as described herein. The kit may comprise containers of substances for reverse transcribing and amplifying one or more miRNAs, such as containers comprising dNTPs (each of the four deoxynucleotides dATP, dCTP, dGTP, and dTTP), buffers, reverse transcriptase and DNA polymerase. The kit may also comprise nucleic acid template(s) for a positive control and/or negative control reaction.

The present invention also relates to the use of the herein described nucleic acid, set of nucleic acids, or composition for the diagnosis of colorectal cancer.

### EXAMPLES

In order to investigate the genome-wide miRNA expression in human colorectal cancer we used the Illumina high throughput sequencing technology. We sequenced and determined the differences of microRNA expression in normal, matching tumor and metastasis-tissues from 8 different colon cancer patients. Altogether we analyzed 24 smallRNA samples and identified several alterations in the miRNA expression patterns.

For each sample we aimed to sequence more than 25 million reads of which 8 million could be aligned uniquely and of which 71,34% were located on miRNA regions as taken from miRBase15. Approximately 730 miRNAs could be detected over all experiments containing the sequencing of patients and cell lines. In regard to the reproducibility of the approach we achieved for technical replicates of SW480 and SW620 cell lines a pearson's correlation coefficient of 0,97 and 0,86, respectively.

In addition, to estimate the reliability of our next generation sequencing results we analyzed the smallRNA fraction of two colorectal cancer cell lines (SW 480 and SW620) in 2 technical replicates. The reproducibility for the technical replicate pairs was very good with a Pearson correlation coefficient of 0.97 for SW480 and 0.86 for SW620. Furthermore, we validated the generated NGS data with TaqMan assays for 4 up-regulated (miR-31, miR-135b, miR-183, miR-96) and 6 down-regulated microRNAs (miR-1, miR-145, miR-133a, miR-133b, miR-150, miR-375) in up to sixteen patients. The miRNAs were selected on the basis of their expression level and on their profile in the literature. The microRNAs miR-31 (FC = 31.02, p-value = 0.003), miR-135b (FC = 21.08, p-value = 0.0001), miR-183 (FC = 7.63, p-value = 0.001) and miR-96 (FC =5.82, p-value = 0.004) are among the top five up-regulated tumor specific candidates. MiR-1 (FC = 13.9, p-value = 0.0004), miR-145 (FC = 2.68, p-value =0.002) are significantly down-regulation in tumor and metastases (p-value < 0.05).

### Biomarkers for diagnosis of colorectal cancer

We identified several miRNAs to be differentially regulated in tumor and metastasis tissue, some of which have already been described in the literature as differentially regulated in cancerous tissues. For microRNA-135b, known in the literature as up-regulated in cancer, we found a 21-fold up-regulation in the colon cancer samples (p-value = 0.0001) and a 28-fold higher expression in metastasis tissue (FC = 28.94, p-value = 0.00156). For microRNA-1 we observed a strong down-regulation in both malignant tissues with more than 10-fold down-regulation in tumor (FC miR-1-1 = 13.9, p-value = 0.00042; FC miR-1-2 = 10.06, p-value = 0.00179) and metastasis tissue (FC miR-1-1 = 18.34, p-value = 0.00409; FC miR-1-2 = 14.12, p-value = 0.00092) in metastases tissue. MicroRNA-133, which is clustered on the same chromosomal locus as microRNA-1 was also found down-regulated. The genome locus of miR-133a-2 which is clustered together with miR-1-1 on chromosome 20 showed a 1.4-fold down-regulation in tumor and a 1.33-fold decreased expression in metastases, whereas the p-value for both expression values were not significant. MiR-133a-1, which is localized in a cluster with miR-1-2 on chromosome 18 is not present in our NGS data set.

Most importantly, we found a number of biomarkers for colorectal cancer having diagnostic capabilities that have previously been not associated with cancer and/or colorectal cancer as listed in tables 1 and 2.

### Biomarkers for differentiation between tumor and metastasis

To identify miRNAs that could play a critical role for the progression of colorectal cancer into metastasis, we compared the expression of miRNAs in the tumor with those in the metastases tissue of each colon cancer patient. We identified a small number of significantly deregulated miRNAs for the metastases. Based on our significance criteria (p-value <0.05) we determined 6 microRNA candidates that were up-regulated in the metastases as compared to the tumor, miR-184 showed the most significant up-regulation of 5-fold (FC = 5.09, p-value = 0.01695).

A higher number of down-regulated miRNAs could be observed for the metastases compared to the tumor tissue. We identified 11 candidates, of which miR-1266 showed the strongest down-regulation of 3.16-fold (p-value = 0.00644).

Overall, we detected an absolute number of 559 miRNAs that were present in all tissues of the patients. Analyzing the intersections of the regulated microRNAs as observed between normal, tumor and metastases tissue, resulted in 19 miRNAs specifically up-regulated in the tumor and 29 miRNAs specifically up-regulated in the metastases when compared to the normal tissue. For the down-regulated miRNAs as compared to the normal tissue, we determined 13 tumor specific and 16 metastases specific miRNAs.

The higher numbers of metastasis-specific regulated miRNAs supports the assumption that the metastasis is a progression of tumor tissue. We identified one microRNA, miR-559, which is down-regulated in all malign tissues as compared to the normal tissue with an even stronger down-regulation in the metastasis as compared to the tumor tissue.

The biomarkers listed in Table 1 have been shown to exhibit differential diagnostic capabilities.

### General tumor biomarkers

We next investigated if it is possible to separate the different tissues based on their miRNA profile. For this, we used principal component analyses as well as unsupervised hierarchical clustering approaches for normal, tumor and metastases tissue of the 8 CRC patients. On the miRNA level we found tumor and metastasis tissues significantly different from normal tissues, but less distinctive between each other. The cluster analysis indicated a strong correlation within the groups of tissues for the expression levels of the candidate microRNAs and it divided the malign and benign colon tissue in two different groups. Tumor and metastasis tissues did not show a separation using absolute expression levels, which could indicate similarity of both malign tissues.

So far we were able to identify malign colon tissues based on a set of 41 miRNAs. However, suggesting miRNAs for a potential clinical application as biomarkers this number is still too high. We therefore asked whether it is possible to narrow down the number of marker miRNAs and found miRNA-1 and miRNA135 to nearly completely separate benign and tumor tissues suggesting that these miRNAs are important deregulators in colon tumors.

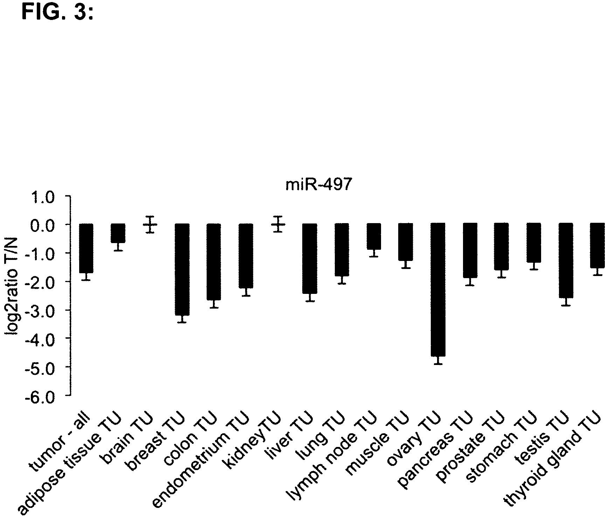

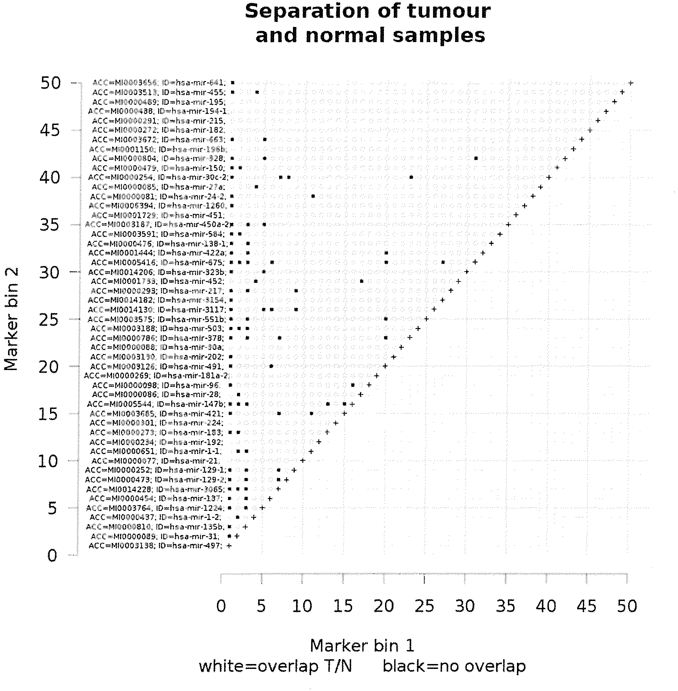

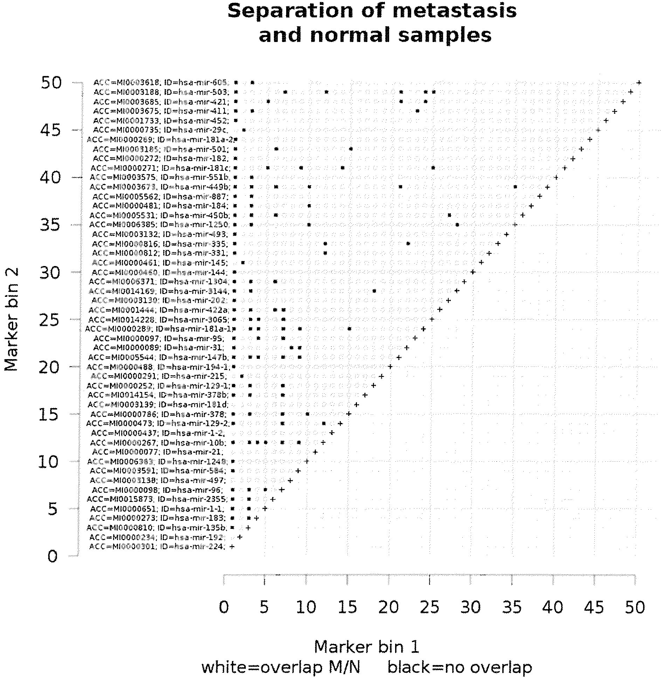

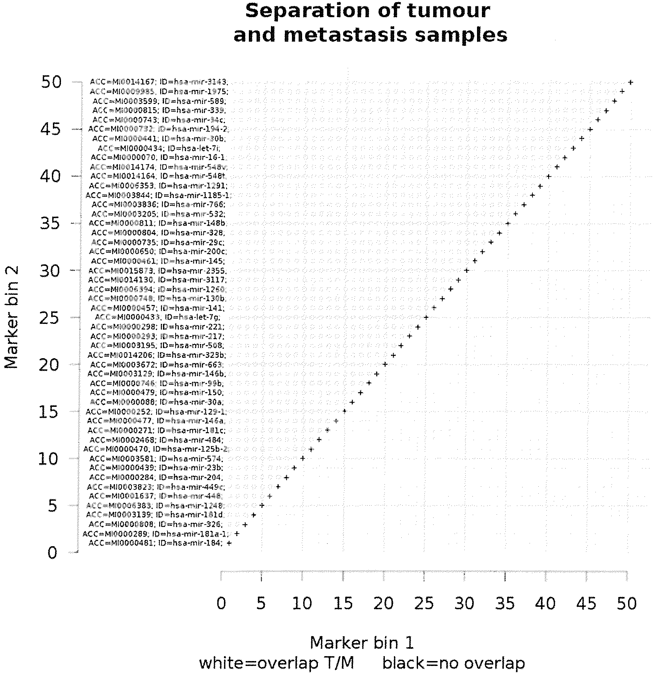

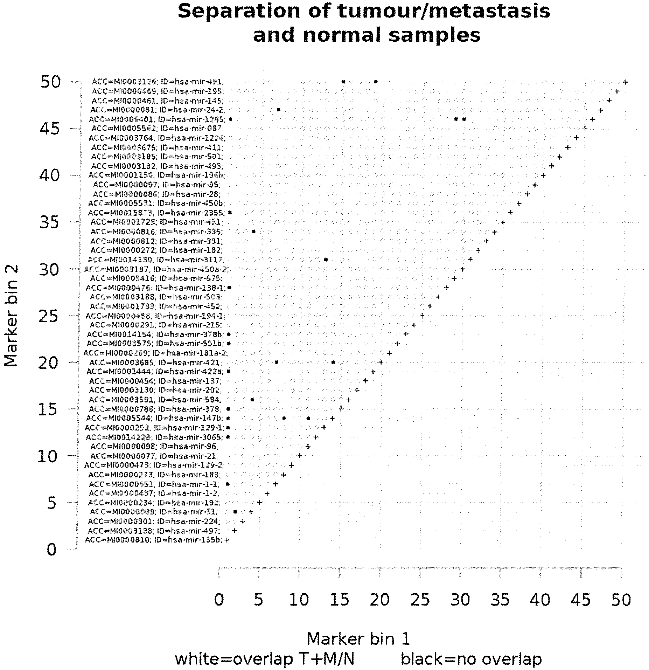

Since miRNA-1 and miRNA-135 are the most significant de-regulated miRNAs in colorectal tumors we asked whether these miRNAs are specific for colorectal tissues or are general tumor suppressors and oncogenes. We thus performed a screen on 15 additional different tumor entities with 320 different tissue samples. For each tumor entity we used normal and tumor tissues (adipose tissue, brain, breast, colon, endometrium, kidney, liver, lung, lymph node, muscle, ovary, pancreas, prostate, stomach, testis and thyroid gland). Here we found that miRNA-1 showed constantly a significantly decreased expression over all tissues. The most significant down-regulations were in muscle (FC = 20.28), ovary (FC = 88.87) and prostate tissues (FC = 45.94). The expression analysis of miRNA-135b delivered a variable pattern across all cancer tissues investigated. In colon cancer we could observe an up-regulation (FC = 9.23) as expected. In endometrium and stomach we estimated a significant up-regulation for the tumor tissue as well (endometrium = 15.44-fold, stomach = 5.7-fold) (Figure 3). However, in 7 tissues (adipose tissue, brain, colon, endometrium, lung, ovary and stomach) we found an increased expression level of miRNA-135b. In the majority of the investigated tissues, such as breast, kidney, liver, lymph node, muscle, pancreas, prostate, testis and thyroid gland miR-135b showed a down-regulation.

MiR-497 has been demonstrated to be suited for diagnosis of cancer in general.

### Discussion

### Analysis and identification of microRNAs as biomarker candidates in colon cancer

Our genome-wide analyses of microRNA expression in colon cancer patients and colon cancer cell lines delivered a range of interesting microRNA candidates, in specific miR-1 and miR-135b, both were top deregulated candidates in tumor and metastases. In contrast to the extremely down-regulated miR-1 we found microRNA-135b up-regulated in the colon tumor tissues. This specific microRNA is already known in the context of CRC. Some authors (Nagel, R. et al. Cancer Res 68, 5795-802 (2008); Bandres, E. et al. Mol Cancer 5, 29 (2006); Wang, Y.X. et al. J Dig Dis 11, 50-4; Necela, B.M., Carr, J.M., Asmann, Y.W. & Thompson, E.A. PLoS One 6, e18501.) revealed a genetic mechanism for the control of the Wnt signaling pathway depending on the expression levels of miR-135b and miR-135a. An up-regulation of these microRNA showed a negatively correlation to the APC expression in vitro. This leads to the assumption that alterations in the miR-135 expression contributes to colorectal cancer pathogenesis. Less is known about the contribution of miR-135b to the progression of metastases. An indication for an involvement could be the even higher expression in the metastases tissue of our CRC patients. Compared the 21-fold up-regulation in the tumor we observed here a significant 28-fold up-regulating over all analyzed patients. This aids the assumption that miR-135b could play a critical role in further stages of colon cancer. Not only miR-135b showed a considerable increased expression in malign tissues, we also determined and validated microRNA candidates, such as miR-31, miR-183 and miR-96. For those candidates literature entries could be found that demonstrate an association between the up-regulation and CRC progression.

By comparing the expression of significantly deregulated microRNAs for the three tissues (normal, tumor, metastases), we identified a total of 29 miRNAs whose expression was down-regulated and 17 miRNAs whose expression was up-regulated in tumor and metastases as compared to the normal tissue. A special microRNA that became highlighted during the intersection analysis was microRNA-559, which effects on the proto-oncogene ERBB2. The ERBB2 or HER2 gene (Human Epidermal growth factor Receptor 2) encodes a member of the epidermal growth factor (EGF) receptor family of receptor tyrosine kinases. The receptors of this family are involved in signal transduction pathways leading to cell growth and differentiation and the overexpression of ERBB2 is decidedly associated to the onset of certain types of cancer including 20-30% of breast and ovarian carcinomas. The human epidermal growth factor receptor 2 is an example for an approved candidate for targeted cancer therapies. Due to the fact, that we found miR-559 constantly and significantly down-regulated in our colon cancer samples and that microRNAs in general become more and more attractive for the development of new therapies that specifically target oncogenes, promoted microRNA-559 to be a promising candidate for further studies and possibly for the development of targeted therapies in colon cancer treatment. The overlap analyses between the various comparisons of normal, tumor and metastases tissue also elucidated interesting microRNA candidates, such as miR-1, miR-135b, miR-129, miR-215, miR-497 and miR-493, which were over both malign tissues significantly deregulated. Those candidates were investigated in further analyses including a microRNA expression screen over different tumor entities.

Analyzing the biomarker candidates we determined that a down-regulation of microRNAs is a more common phenomenon than an up-regulation of microRNAs in different cancer tissues, which is more heterogeneous. In general there is a trend of microRNA down-regulation reported in different studies, for example, by Lu et al. in 2005 (Lu, J. et al. Nature 435, 834-8 (2005)), in connection with different human cancer entities. These findings lead to the assumption, that major components of the microRNA biogenesis machinery, like DICER might be deregulated in various cancers. The inhibition of DICER machinery could be an initial event for the onset of cancer considering the fact that a general down-regulation of DICER should contribute an inevitably reduction of mature microRNA biogenesis. The abated regulation of down-stream targets, such as oncogenes could lead to their up-regulation.

For an additional microRNA we could observe a down-regulation over the unity of all tumor samples including microRNA screening experiments. MicroRNA-215 is already described in the context of colon cancer by Georges et al. (Georges, S.A. et al. Cancer Res 68, 10105-12 (2008)) and Braun et al. (Braun, C.J. et al. Cancer Res 68, 10094-104 (2008)), in 2008 and Song et al. (Song, B. et al. Mol Cancer 9, 96 (2010)) in 2010. They described the correlation between miR-215 (which is arranged in a cluster with miR-192 and miR-194) and a cell cycle arrest at G2-phase due to a p53-dependent up-regulation of p21 in colon cancer cells.

To assess the possibility of selected microRNA candidates to function as biomarkers for clinical applications was one of the major questions of this work. For the set of differentially expressed microRNAs we performed PCA analyses and hierarchical clustering and achieved a distinct separation of malign and benign tissue. On a smaller set of microRNAs (miR-1, miR-135b, miR-129, miR-493*, miR-215 and miR-497) we applied PCA analyses on different cancer entities and for brain, breast, colon, kidney, muscle, prostate, stomach, testis and thyroid gland we separated tumor from normal tissue. Narrowing down the set of microRNAs that open a chance for clinical applications we investigated miR-1 and miR-135b, our top candidates, and achieved a nearly full parting of tumor and normal tissue for colon cancer. Altogether our data suggest and support the relevance of our selected miRNA candidates to function as markers for cancer diagnosis and they provide the potential to deliver novel therapeutic targets, not only for colon cancer. To find candidates that indicate the development of the tumor into distant sites are more delicate. This could be caused by the similarity of both tissues.

### Material and Methods

### Colorectal cancer tissue samples and cell lines for NGS experiments

The primary colon carcinoma tissue and matched normal colonic epithelium as well as liver metastases tissue used for the NGS experiments were obtained from patients diagnosed with CRC and undergoing surgical resection. The samples were snap frozen immediately and stored at -80°C.

Colon cancer cell lines SW480 and SW620 were cultured in a humified atmosphere of 90% air, 5% CO₂ using Dulbecco's Modified Eagle's Medium (DMEM) medium and 10% fetal calf serum (FCS) and 1% penicillin/streptomycin.

### RNA isolation from human tissue samples ad cell lines

RNA was extracted applying Trizol (Invitrogen) reagent according to the manufacture's protocol and the RNA integrity was assessed using the Agilent BioAnalyzer 2100 technology (Agilent).

### Tissue samples for cancer screen

In total 243 tumor samples and 87 normal tissue samples were selected from a tissue bank established at the Institute of Pathology (Medical University of Graz, Austria) to estimate the microRNA expression in a variety of human cancer types. This archive consists of tumor and normal tissue samples obtained from different organs, such as adipose tissue, brain, breast, colon, endometrium, kidney, liver, lung, lymphatic system, muscle, ovary, pancreas, prostate, stomach, testis and thyroid gland. All samples were reviewed and diagnosed histopathologically and RNA quality was assessed by automated electrophoresis.

### SmallRNA library preparation and Solexa Sequencing

SmallRNA preparation from human colon tissue and cell lines for Solexa sequencing included the following steps: smallRNA isolation, cDNA library preparation, sequencing and was performed by Illumina's DGE smallRNA sample prep kit following the manufacture's instructions. In short 10µg total RNA was size-fractionated on a denaturing 15% TBE-urea polyamide gel and smallRNA fragments of 20-30 bases in length were purified. To generate cDNA the Illumina 3' adapter (sequence) and 5' adapter (sequence) were ligated to the size selected RNA molecules to enable reverse transcription and an amplification step as well as the sequencing procedure. To remove unligated adapters, the ligation products of the desired size range between 70-90 bases in length were purified from a 10% TBE-urea polyacrylamide gel. The precipitated RNA was converted to single-stranded cDNA using Superscript II revese transcriptase (Invitrogen) and Illumina's smallRNA RT-Primer (sequence). Followed by a PCR-amplification step in 15 cycles using Phusion DNA Polymerase and Illumina's small RNA primer set (sequences) the amplification products were seperated on a 6% TBE polyacrylamide gel. The corresponding gel fragment of approximately 92 bases in length was excised. The purified DNA was quantified and diluted to 10nM for cluster generation and sequencing on Illumina Genome Analyzer.

### Analysis of deep sequencing data

### Primary data analysis

Sequences were obtained using Illumina Genome Analyser IIx and images from the instrument were processed using the manufacturer's software to generate FASTQ sequence files. All reads were mapped against Illumina adaptor sequences using blat [PMID 11932250] and adaptor signatures were clipped from the reads subsequently. In detail; clipping always starts from one of the ends of a read and reads with a length less than 16 bp after clipping were omitted from analysis.

### Analysis of miRNA expression

Adaptor-clipped reads were mapped to the human genome version GRCh37 (hg19) with the bwa 0.5.8 alignment tool [PMID 19451168] using default parameters. As a measure for miRNA expression, reads on target regions were counted. A read had to have at least one base within the target region to be evaluated "on target". Log2 ratios were calculated using the read counts per target to compare different conditions. Ratios were subsequently normalized for each comparison by centering the median of log2 ratios to zero. Differential expression was evaluated using student's t-test on the log2 ratios (samples versus controls).

To validate the miRNA expression analysis strategy we compared sequencing data to TaqMan data for 10 different miRNAs in up to 8 patients: Normalized log2 ratios from both datasets were plotted against each other and a linear model (y = mx + b) was fitted for two comparisons: tumor vs benign and metastasis vs benign. We calculated the *Pearson* product-moment *correlation for both comparisons* to demonstrate the agreement of both datasets.

### Analysis of mRNA expression

Adaptor-clipped reads were mapped to the human genome version GRCh37 (hg19) using transcript models taken from Ensembl v64 with TopHat [PMID 19289445] using the parameters -u -N --max-bundle-length 350000000 --max-bundle-frags 50000000. Differential expression as well as unknown transcript models were determined using the Cufflinks software bundle [PMID 21697122]. In detail transcript models from all tissues were merged for each patient using cuffcompare and differential expression was determined using cuffdiff with upper quartile normalization.

### Real-time quantification of microRNAs using stem-loop real-time PCR

For real-time quantification of mature microRNAs we used TaqMan® MicroRNA Assays (Applied Biosystems) and performed a two-step RT-PCR according to the manufacturer's protocol.

In the reverse transcription reaction, single-stranded cDNA was synthesized from 25ng total RNA using 50nM looped RT primer (PN 4427975; has-miR-1, has-miR-135b, has-miR-215, has-miR-493*, has-miR-497,..., has-miR-129-3p, Assay ID: 001184 and has-miR-9, Assay ID: 000583), 1X Reverse Transcription buffer, 0,25mM each of dNTPs, 0,25U/µl RNase Inhibitor and 3,33U/µl MultiScribe™ Reverse Transcription per 15µl reaction (TaqMan® MicroRNA Reverse Transcription Kit, Applied Biosystems PN 4366596). The reaction was incubated in a thermal cycler (Applied Biosystems 9700 Thermocycler) for 30 min at 16°C, 30 min at 42°C, followed by 5 min incubation at 85°C to inactivate the Multiscribe Reverse Transcriptase and then hold at 4°C. The cDNA was diluted 10-fold before preparing for PCR reaction.

For the quantification of the RT-products TaqMan® minor groove binding (MGB) probes were used in a 10µl PCR reaction, containing 1µl of the 10-fold diluted cDNA, 1X TaqMan® MicroRNA Assay (PCR Primer/Probe/dNTP mix) and 1X TaqMan® Universal PCR master mix. The real-time PCR was performed in a 384-well plate using standard protocols on an Applied Biosystems 7900HT Fast Real-Time PCR System, including 10 min incubation at 95°C to activate the AmpliTaq® Gold enzyme, followed by 40 amplification cycles of 95°C for 15 s and 60°C for 1 min. All reactions were typically run in triplicates.

### Real-time quantification of mRNA targets

Total RNA from cells was isolated using Trizol reagent according to the manufactures protocol. In the following step, DNA was digested using RNase-free DNase kit (Qiagen) and cDNA was synthesized from total RNA using gene specific primers for miR-1 target genes. The concentration of the cDNA was quantified using NanoDrop Spectophotometer.

### Data analysis for real-time quantification

The relative quantification of microRNA and mRNA expression was calculated using the comparative "delta CT" method. Default threshold settings were applied to determine the Ct value, which is defined as the fractional cycle number at which the fluorescence passes the fixed threshold.

### FIGURE CAPTIONS

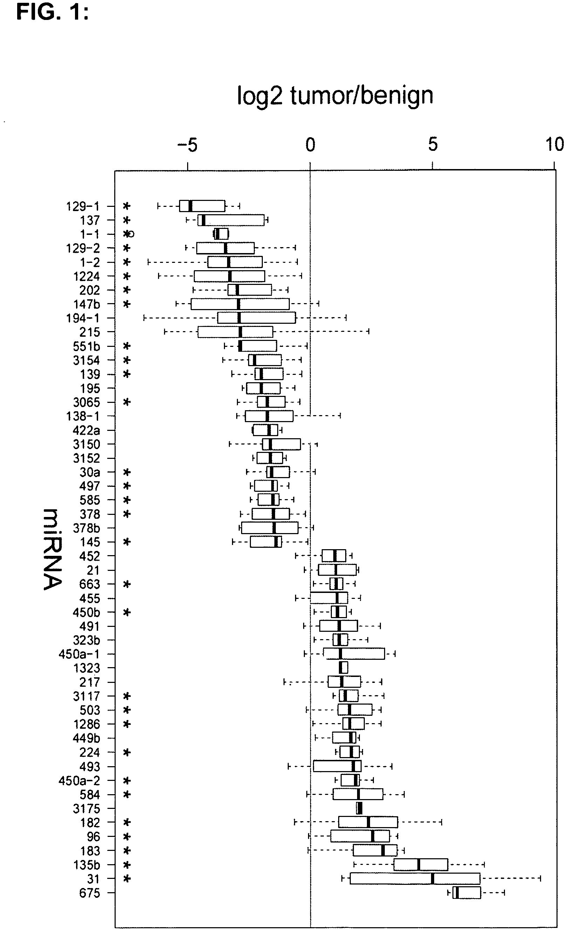

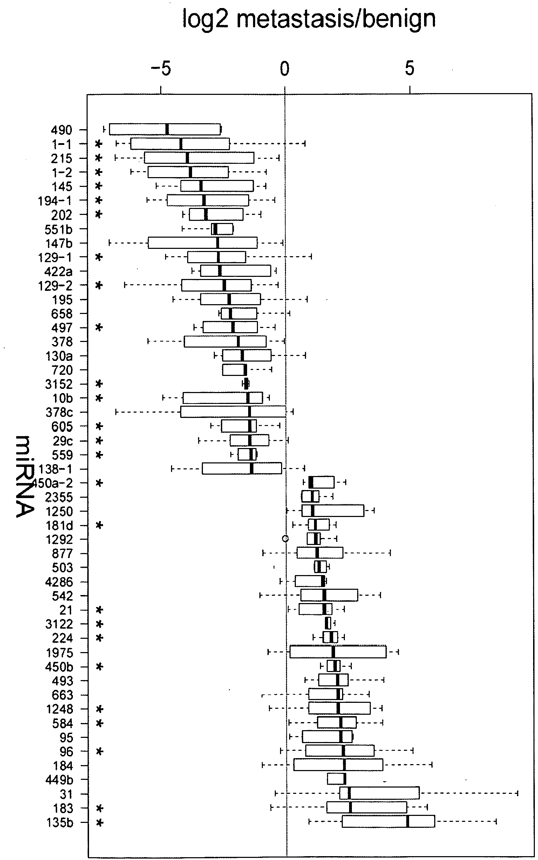

**FIG. 1A****: Differential expression of miRNAs in colon tumor tissues. Significant top 25** up- and down-regulated miRNAs comparing tumor versus normal colon samples, sorted by median log2ratio. Analysis of 16 colorectal cancer samples (normal and tumor tissue) using NGS technology delivered a range of tumor specific deregulated miRNAs. All depicted miRNAs sufficed a p-value threshold ≤ 0.05. A star indicates samples with a p-value of below 0.01.
**FIG. 1B****: Differential expression of miRNAs in colon metastasis tissues. Significant top** 25 up- and down-regulated miRNAs comparing metastasis versus normal colon samples, sorted by median log2ratio. Analysis of 16 colorectal cancer samples (normal and tumor tissue) using NGS technology delivered a range of tumor specific deregulated miRNAs. All depicted miRNAs sufficed a p-value threshold ≤ 0.05. A star indicates samples with a p-value of below ≤0.01.

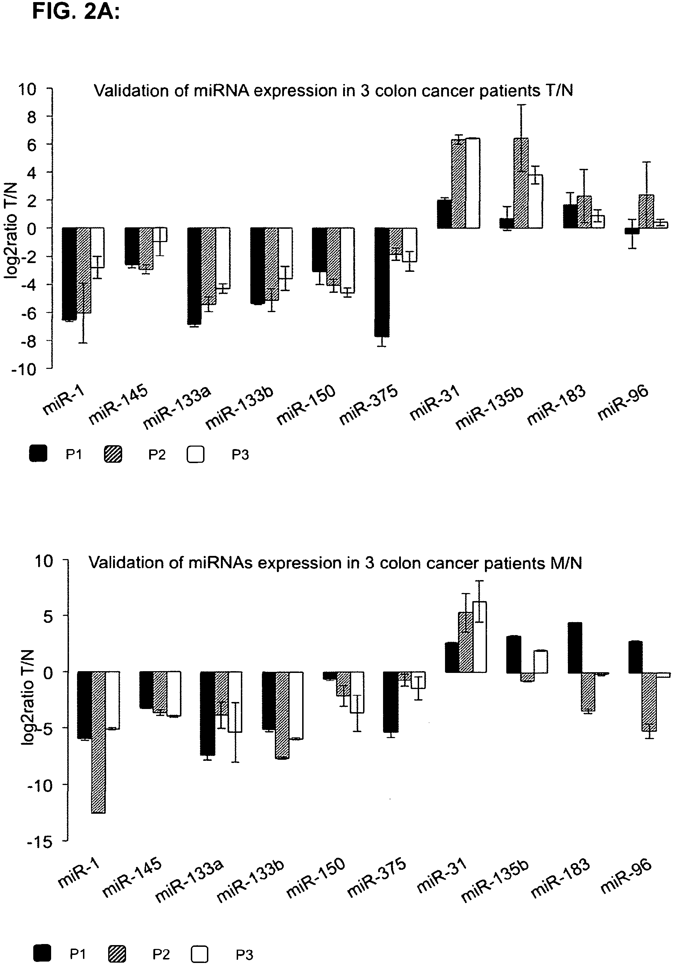

**FIG. 2A****:** Upper graph: Validation of 10 microRNA expression pattern in tumor vs normal tissue for 3 colon cancer patients. Lower graph: Validation of 10 microRNA expression pattern in metastases vs normal tissue for 3 colon cancer patients.

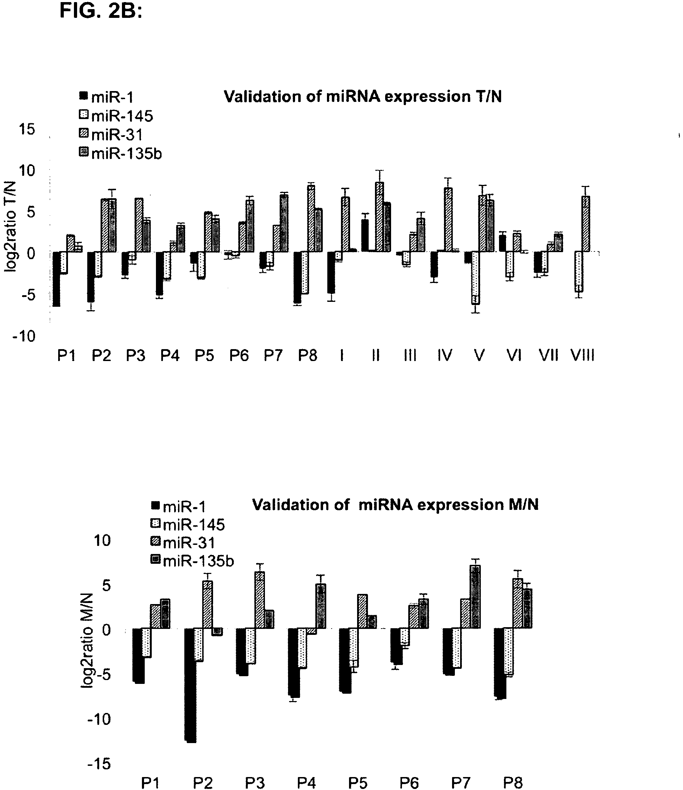

**FIG. 2B****:** Upper graph: Validation of miR-1, miR-135b, miR-145 and miR-31 expression in tumor vs normal tissue for 16 colon cancer patients. Lower graph: Validation of miR-1, miR-135b, miR-145 and miR-31 expression in metastases vs normal tissue for 8 colon cancer patients
**FIG. 3****:** Down-regulation of miR-497 in 15 additional tumor entities. QPCRs for miR-497 were performed in 330 additional tumor and normal samples and ratios were calculated between normal and tumor.
**FIG. 4** is an exemplary diagram of a computing device comprising a client and/or server according to an implementation consistent with the principles of the invention.
**FIG. 5** is a flowchart of exemplary processing of methylation status for biomarker(s) present in biological samples according to an implementation consistent with the principles of the present invention.

## Claims

1. A method for differential diagnosis of colon cancer in a patient, comprising the following steps of,
a. determining in a sample of a subject the amount of one or more of the micro RNAs (miRNA) selected from the list of Table 1,
b. comparing the amount of miRNA determined to a predetermined reference value,
c. wherein if the miRNA is, in comparison to said reference value, either down-regulated or up-regulated as designated in Table 1, the sample is designated as metastatic.
**Table 1:**
| microRNA name | regulation in metastatic sample as compared to reference value |
|---|---|
| miR-194-1 | down-regulated |
| miR-202 | down-regulated |
| miR-551b | down-regulated |
| miR-129-2 | down-regulated |
| miR-658 | down-regulated |
| miR-497 | down-regulated |
| miR-3152 | down-regulated |
| miR-378c | down-regulated |
| miR-605 | down-regulated |
| miR-559 | down-regulated |
| miR-184 | up-regulated |
| miR-1248 | up-regulated |
| miR-450b | up-regulated |
| miR-1975 | up-regulated |
| miR-3122 | up-regulated |
| miR-542 | up-regulated |
| miR-4286 | up-regulated |
| miR-503 | up-regulated |
| miR-877 | up-regulated |
| miR-1292 | up-regulated |

2. The method according to claim 1, wherein the predetermined reference value represents the amount of said miRNA present in tumor or benign tissue.

3. A method for diagnosis of colon cancer in a patient, comprising the following steps of,
a. determining in a sample of a subject the amount of one or more of the micro RNAs (miRNA) selected from the list of Table 2,
b. comparing the amount of miRNA determined to a predetermined reference value,
c. wherein if the miRNA is, in comparison to said reference value, either down-regulated or up-regulated as designated in Table 1, the sample is designated as cancerous
**Table 2:**
| microRNA name | regulation in cancerous sample as compared to reference value |
|---|---|
| Hsa-mir-1224 | down-regulated |
| Hsa-mir-202 | down-regulated |
| Hsa-mir-147b | down-regulated |
| Hsa-mir-3154 | down-regulated |
| Hsa-mir-3065 | down-regulated |
| Hsa-mir-3150 | down-regulated |
| Hsa-mir-3152 | down-regulated |
| Hsa-mir-497 | down-regulated |
| Hsa-mir-585 | down-regulated |
| Hsa-mir-378b | down-regulated |
| Hsa-mir-3175 | up-regulated |
| Hsa-mir-450a2 | up-regulated |
| Hsa-mir-449b | up-regulated |
| Hsa-mir-1286 | up-regulated |
| Hsa-mir-3117 | up-regulated |
| Hsa-mir-1323 | up-regulated |
| Hsa-mir-450a-1 | up-regulated |
| Hsa-mir-323b | up-regulated |
| Hsa-mir-450b | up-regulated |
| Hsa-mir-452 | up-regulated |

4. The method according to claim 3, wherein the predetermined reference value represents the amount of said miRNA present in benign tissue.

5. The method according to any one of the preceding claims, wherein the amount of miRNA is determined by using miRNA array techniques or RT-PCR.

6. The method according to any one of the preceding claims, wherein the sample originates from blood, urine, semen, preferably from colorectal secretions, isolated colorectal cells, and most preferably from colorectal tissue.

7. The method according to any of the preceding claims, wherein the determining step is conducted by a computing device.

8. The method according to any of the preceding claims, wherein the comparison step is conducted by a computing device.

9. The method according to any of the preceding claims, further comprising outputting for presentation on a display associated with the computing device.

10. A nucleic acid that is capable to specifically detect one of the miRNAs of claims 1 or 3.

11. The nucleic acid according to claim 10, wherein the nucleic acid is 15 to 30 nt in length.

12. The nucleic acid according to any of the claims 10 to 11, wherein the nucleic acid is a primer or a probe.

13. The nucleic acid according to claim 12, wherein the probe is labelled, preferably, wherein the probe is a TaqMan probe.

14. A set of nucleic acids according to any of the claims 10 to 13 for use in multiplex RT-PCR or in microarray techniques.

15. A composition for the diagnosis of colorectal cancer comprising a nucleic acid according to any of the claims 10 to 13, or a set of nucleic acids according to claim 14.

16. A kit for the diagnosis of colorectal cancer comprising a nucleic acid according to any of the claims 10 to 13, or a set of nucleic acids according to claim 14, or a composition according to claim 15.

17. Use of the nucleic acid of any of the claims 10 to 13, or of the set of nucleic acids according to claim 14, or a composition according to claim 15 for the diagnosis or differential diagnosis of colorectal cancer.
